(19) Europäisches Patentamt
European Patent Office
Office européen des brevets

(11) **EP 1 230 932 B1**

(12) **EUROPÄISCHE PATENTSCHRIFT**

(45) Veröffentlichungstag und Bekanntmachung des Hinweises auf die Patenterteilung:
**12.01.2005 Patentblatt 2005/02**

(51) Int Cl.⁷: **A61K 39/395**, A61K 39/39,
A61P 35/00
// C07K16/30

(21) Anmeldenummer: **02005394.8**

(22) Anmeldetag: **12.01.2000**

(54) **Verwendung von Antikörpern zur Vakzinierung gegen Krebs**

Use of antibodies for anticancer vaccination

Utilisation d'anticorps pour effectuer une vaccination contre le cancer

(84) Benannte Vertragsstaaten:
**AT BE CH CY DE DK ES FI FR GB GR IE IT LI LU MC NL PT SE**
Benannte Erstreckungsstaaten:
**LT LV MK SI**

(30) Priorität: **13.01.1999 CH 5199**

(43) Veröffentlichungstag der Anmeldung:
**14.08.2002 Patentblatt 2002/33**

(62) Dokumentnummer(n) der früheren Anmeldung(en) nach Art. 76 EPÜ:
**00906191.2 / 1 140 168**

(73) Patentinhaber: **Igeneon Krebs-Immuntherapie Forschungs- und Entwicklungs-AG**
**1230 Wien (AT)**

(72) Erfinder:
 • **Eckert, Helmut**
  **4104 Oberwil (CH)**
 • **Loibner, Hans**
  **1238 Wien (AT)**

(74) Vertreter: **VOSSIUS & PARTNER**
**Siebertstrasse 4**
**81675 München (DE)**

(56) Entgegenhaltungen:
WO-A-96/01126        WO-A-97/15597
WO-A-98/56416        WO-A-98/56419
WO-A-99/65523

 • LEVEUGLE B ET AL: "PSA-directed immunotherapy of prostate cancer" PROCEEDINGS OF THE ANNUAL MEETING OF THE AMERICAN ASSOCIATION FOR CANCER RESEARCH, März 1998 (1998-03), XP002117652
 • PERVIN S ET AL: "Proliferation of T-cells from colon cancer patients by peptides based on the structure of an anti-idiotype antibody mimicking CEA" PROCEEDINGS OF THE ANNUAL MEETING OF THE AMERICAN ASSOCIATION FOR CANCER RESEARCH,US,PHILADELPHIA, AACR, Bd. MEETING 87, 1996, Seite 473 XP002076985 ISSN: 0197-016X
 • CHAKRABORTY M ET AL: "Immune responses in advanced breast cancer patients treated with an anti-idiotype antibody vaccine" PROCEEDINGS OF THE ANNUAL MEETING OF THE AMERICAN ASSOCIATION FOR CANCER RESEARCH,US,PHILADELPHIA, AACR, Bd. 38, 1997, Seite 616 XP002076982
 • DATABASE BIOSIS [Online] BIOSCIENCES INFORMATION SERVICE, PHILADELPHIA, PA, US; Oktober 1997 (1997-10) KATAYAMA MASAHIRO ET AL: "Expression of neural cell adhesion molecule L1 in human lung cancer cell lines." Database accession no. PREV199800073279 XP002255877 & CELL STRUCTURE AND FUNCTION, Bd. 22, Nr. 5, Oktober 1997 (1997-10), Seiten 511-516, ISSN: 0386-7196

**Beschreibung**

Die vorliegende Erfindung betrifft Antikörpern, die gegen das menschliche zelluläre Membranantigen Ep-CAM gerichtet sind, als Vakzine gegen Krebs.

Mit der Entdeckung der Hybridomtechnologie ist es möglich geworden, monoclonale Antikörper (MAK) gegen verschiedenste Antigene zu generieren. Diese generell für alle biologischen Fragestellungen einsetzbare Technologie hat auch in der Krebsforschung einen wichtigen Platz eingenommen. In den letzten 20 Jahren sind MAK gegen eine Vielzahl von Tumor-assoziierten Antigenen (TAA) hergestellt worden. TAA sind Strukturen, die bevorzugt auf der Zellmembran von Tumorzellen exprimiert sind, dadurch eine Unterscheidung zu nicht-malignem Gewebe ermöglichen und daher als Zielpunkte für diagnostische und therapeutische Anwendungen auf der Basis von spezifischen MAK oder davon abgeleiteten Derivaten gesehen werden.

Direkte therapeutische Anwendungen von MAK gegen TAA beruhen auf passiven Immuntherapien, das heißt, ein MAK oder ein Derivat wird systemisch in geeigneter Menge an Krebspatienten verabreicht und übt eine therapeutische Wirkung nur aus, solange die Konzentration im Organismus dafür genügend groß ist. Die biologische Halbwertszeit solcher Agentien hängt von ihrer Struktur ab und beträgt wenige Stunden bis mehrere Tage. Daher ist es notwendig, wiederholte Applikationen vorzunehmen. Das führt bei Verwendung von xenogenen Antikörpern (z.B. murine MAK) aber zu unerwünschten Immunreaktionen, die eine mögliche therapeutische Wirkung neutralisieren und gefährliche Nebenwirkungen (anaphylaktische Reaktionen) bedingen können. Daher können solche Immuntherapeutika nur für eine begrenzte Zeit verabreicht werden.

Einem anderen Ansatz für Immuntherapie von Krebs liegt die selektive Aktivierung des Immunsystems von Krebspatienten zugrunde, maligne Zellen zu bekämpfen. Das wird mittels verschiedenster Formen von Krebsvakzinen versucht. Dazu gehören Impfungen mit autologen oder allogenen Tumorzellen, chemisch oder molekularbiologisch modifizierten autologen oder allogenen Tumorzellen, isolierten oder mit Hilfe von chemischen oder molekularbiologischen Methoden hergestellten TAA, daraus abgeleiteten Peptiden, neuerdings auch Impfungen mit DNA, die für TAA oder daraus abgeleiteten Strukturen codieren, etc. Eine alternative Methode beruht auf der Verwendung von anti-idiotypischen Antikörpern zur Vakzinierung gegen Krebs. Geeignete anti-idiotypische Antikörper können ein TAA immunologisch nachahmen. Sie induzieren als Fremdproteine (z.B. murine Antikörper, Ziegen-Antikörper etc.) nach Vakzinierung - im Gegensatz zu den eigentlichen menschlichen Tumorantigenen, die als Selbst-Strukturen oft nur wenig immunogen sind - im Menschen eine starke Immunantwort. Daher können anti-idiotypische Antikörper als immunogener Ersatz eines Tumorantigens zur Impfung verwendet werden.

Im Unterschied zur passiven Immuntherapien mit Anü-Tumorantikörpem genügen für die aktive spezifische Krebs-Immuntherapie im Prinzip sehr kleine Mengen eines geeigneten Impfstoffes, um für Monate bis Jahre eine Immunität zu induzieren, die oei Abschwächung durch Booster-Impfungen wieder aufgefrischt werden kann. Darüberhinaus kann bei aktiver Immunisierung sowohl eine humorale als auch eine zelluläre Immunität induziert werden, deren Zusammenspiel eine effektive Schutzwirkung ergeben kann.

Zusammenfassend beruht die bisherige Verwendung von Antikörpern oder von deren Derivaten in der Krebs-Immuntherapie im wesentlichen auf zwei Prinzipien:

- Passive Therapie mit Antikörpern oder deren Derivaten, die gegen TAA gerichtetet sind.
- Aktive Immunisierung (Vakzinierung) mit Antikörpern oder deren Derivaten, die gegen den Idiotyp von Antikörpern mit Spezifität gegen TAA gerichtet sind.

Im Verlauf der Entdeckung und nachfolgender Charakterisierung von verschiedensten TAA hat sich herausgestellt, daß diese wichtige Funktionen für Krebszellen haben. Sie ermöglichen den entarteten Zellen, charakteristische Eigenschaften für den malignen Phänotyp wie z.B. vermehrte Adhäsionsfähigkeit auszuüben, die für die Etablierung von Metastasen von großer Bedeutung sind. Allerdings können solche Antigene durchaus in bestimmten Stadien auch auf normalen Zellen exprimiert sein, wo sie für normale Funktionen dieser Zellen verantwortlich sind. Ohne Anspruch auf Vollständigkeit seien hier einige Beispiele für solche Antigene angeführt:

- N-CAM (Neuronal Cell Adhesion Molecule), das oft auf Tumoren neuronalen Ursprungs exprimiert ist und homophile Adhäsion bewirkt (J.Cell Biol. 118 (1992), 937).
- Das Lewis Y Kohlenhydratantigen, das auf der Mehrzahl der Tumoren epithelialen Ursprungs aufscheint, aber auch während der fötalen Entwicklung epithelialer Gewebe eine wichtige Rolle spielt. Es wurde gezeigt, daß die Expression dieses Antigens in Lungenkrebs stark mit einer ungünstigen Prognose assoziiert ist, da Lewis Y positive Krebszellen offensichtlich ein höheres metastatisches Potential haben (N. Engl. J. Med. 327 (1992), 14).
- CEA (Carcino Embryonic Antigen), das häufig auf epithelialen Tumoren des Gastro-Intesfinaltraktes vorkommt und als Selbstadhäsionsmolekül identifiziert wurde (Cell 57 (1989), 327).
- Ep-CAM (Epithelial Cell Adhesion Molecule), daß auf fast allen Tumoren epithelialen Ursprungs exprimiert ist, aber auch auf vielen normalen Epithelien vorkommt, das als Selbstadhäsionsmolekül charakterisiert wurde und daher als pan-epitheliales Adhäsionsantigen klassifiziert werden kann (J. Cell Biol.125 (1994), 437).

Der vorliegenden Erfindung liegt nun die Aufgabe zugrunde weitere Mittel und Methoden zur Verfügung zu stellen, die eine effiziente Prophylaxe gegen bzw. Therapie von Krebserkrankungen erlauben.

Diese Aufgabe wird durch die Bereitstellung der in den Ansprüchen bezeichneten Ausführungsformen gelöst.

Somit betrifft die Erfindung Antikörper, die gegen das menschliche zelluläre Membranantigene Ep-CAM gerichtet sind, als prophylaktische und/oder therapeutische Vakzine gegen Krebs.

Der Begriff "zelluläre Membranantigene" betrifft dabei Strukturen, die auf der Zellmembran von Zellen präsentiert werden. Hier sind insbesondere Rezeptoren zu nennen, wie z.B. der Transferrin-Rezeptor, oder andere Moleküle, wie z.B. E-Cadherin oder Ep-CAM.

Unter dem Begriff "Tumorassoziiertes Antigen" wird hier eine Struktur verstanden, die bevorzugt von Tumorzellen präsentiert wird und dadurch eine Unterscheidung zu nicht-malignem Gewebe ermöglicht. Vorzugsweise ist ein solches Tumor-assoziiertes Antigen auf bzw. in der Zellmembran einer Tumorzelle lokalisiert. Dabei ist aber nicht ausgeschlossen, daß derartige Antigene auch auf nicht-entarteten Zellen vorkommen. Bei Tumor-assoziierten Antigenen kann es sich beispielsweise um Polypeptide, insbesondere glykosylierte Proteine, oder Glykosylierungsmuster von Polypeptiden handeln. Andere Strukturen, die ein Tumor-assoziiertes Antigen darstellen können, sind z.B. Glykolipide. Hierzu gehören beispielsweise Ganglioside, wie z.B. GM2. Ferner können Tumor-assoziierte Antigene dargestellt werden durch Veränderungen der Zusammensetzung von Lipiden der Zellmembran, die für Krebszellen charakteristisch sein können.

Beispiele für Tumor-assoziierte Antigene sind N-CAM, das Lewis Y Kohlenhydratantigen, CEA und Ep-CAM, die bereits oben erwähnt wurden. Weitere Beispiele sind Sialyl Tn Kohlenhydrat, Globo H Kohlenhydrat, Ganglioside wie GD2/GD3/GM2, Prostate Specific Antigen (PSA), CA 125, CA 19-9, CA 15-3, TAG-72, EGF Rezeptor, Her2/Neu Rezeptor, p97, CD20 und CD21. Gegen alle diese Antigene stehen monoclonale Antikörper zur Verfügung. Weitere Tumor-assoziierte Antigene werden z.B. beschrieben in DeVita et al. (Eds., "Biological Therapy of Cancer", 2. Aufl., Kapitel 3: Biology of Tumor Antigens, Lippincott Company, ISBN 0-397-51416-6 (1995)).

Unter dem Begriff "Antikörper" werden Antikörper aller Art verstanden, insbesondere polycfonale oder monoclonale oder auch chemisch, biochemisch oder molekularbiologisch hergestellte Antikörper. Verfahren zur Herstellung solcher Moleküle sind dem Fachmann geläufig. Auf die Art der Herstellung des Antikörpers kommt es nicht an. Wichtig ist lediglich seine Bindungsspezifität für ein relevantes Epitop eines zellulären Membranantigens. Bevorzugt werden monoclonale Antikörper verwendet, ganz besonders bevorzugt monoclonale Antikörper tierischen, insbesondere murinen Ursprungs. Ganz besonders bevorzugt wird der murine monoclonale Antikörper HE-2 verwendet, der wie beschrieben erzeugt werden kann oder ein Antikörper, der die gleiche Bindungsfeinspezifität wie HE2 aufweist. Der Begriff "Antikörper" umfaßt im Rahmen der vorliegenden Erfindung auch Fragmente und Derivate von Antikörpern, wobei diese Fragmente oder Derivate ein TAA erkennen. Die durch Vakzinierung mit geeigneten Antikörpern gegen TAA induzierte, therapeutisch wirksame Immunantwort ist durch die Bindungsregion dieser Antikörper, ihrem Idiotyp, determiniert. Daher können im Prinzip statt intakter Antikörper auch Fragmente oder Derivate dieser Antikörper erfolgreich zur Vakzinierung eingesetzt werden, solange diese Derivate den idiotyp des jeweiligen Ausgangs-Antikörpers weiterhin beinhalten. Als Beispiele, jedoch nicht auf diese eingeschränkt, seien erwähnt: F(ab)'$_2$-Fragmente, F(ab)'-Fragmente, Fv-Fragmente, die entweder nach an sich bekannten biochemischen Methoden (enzymatische Spaltung) oder nach an sich bekannten Methoden der Molekularbiologie hergestellt werden können, sowie Antikörperderivate, die nach an sich bekannten chemischen, biochemischen oder molekularbiologischen Methoden hergestellt werden können. Der Begriff Derivat umfaßt dabei insbesondere auch Produkte, die erzeugt werden können durch chemische Kopplung von Antikörpern (-fragmenten) mit Molekülen, die die Immunantwort verstärken können, wie z.B. Tetanus-Toxoid, Pseudomonas Exotoxin, Derivate von Lipid A, GM-CSF, IL-2 oder durch chemische Kopplung von Antikörpern (-fragmenten) mit Lipiden für bessere Inkorporierung in eine Liposomenformullerung. Der Begriff "Derivat" umfaßt auch molekularbiologisch hergestellte Fusionsproteine von Antikörpern (-fragmenten) mit Polypeptiden, die die Immunantwort verstärken können, wie GM-CSF, IL-2, IL-12, C3d etc. Die Antikörper können erfindungsgemäß selbstverständlich auch in Kombination eingesetzt werden. D.h. es können zwei oder mehr Antikörper, die verschiedene Membranantigene oder verschiedene Epitope desselben Membranantigens erkennen verabreicht werden. Dabei können die verschiedenen Antikörper jeweils gleichzeitig (gemeinsam oder getrennt) oder nacheinander verabreicht werden. Krebszellen exprimieren häufig mehrere TAA gleichzeitig, gegen die für Vakzinierungen geeignete Antikörper zur Verfügung stehen oder generiert werden können. Für eine verstärkte, unter Umständen synergistische Wirkung der induzierten Immunantwort, um die potentielle Gefahr der Selektion von Antigennegativen Varianten zu minimieren und um möglicher Tumorzellheterogenität entgegenzuwirken, kann es günstig sein, eine Kombination von zwei oder mehreren geeigneten Antikörpern oder deren Fragmenten oder Derivaten gleichzeitig zur Vakzinierung zu verwenden.

Der Begriff "Vakzinierung" bedeutet im Rahmen der vorliegenden Erfindung eine aktive Immunisierung, d.h. eine Induktion einer spezifischen Immunantwort durch Verabreichung (z.B. subkutan, intradermal, intramuskulär, ev.

auch oral, nasal) von geringen Mengen eines Antigens (eines Stoffes, der vom Immunsystem des vakzinierten Individuums als fremd und damit immunogen erkannt wird) in einer geeigneten immunogenen Formulierung. Das Antigen wird also als "Trigger" für das Immunsystem benutzt, um eine für das Antigen spezifische Immunantwort aufzubauen. Die dazu benötigten Mengen des Antigens können grundsätzlich sehr gering sein (manche Impfstoffe beinhalten nur ca. 5-10 µg Antigen pro Impfdosis). Charakteristisch für eine aktive Immunisierung ist eine in großen Bereichen nur wenig Mengen-abhängige Dosis Wirkungskurve. Das heißt, daß die Immunantwort in einem weiten Dosisbereich in etwa gleich stark ausfällt. Daraus folgt, daß bei einer Vakzinierung der gewünschte Effekt, also die Induktion einer Immunantwort, bereits mit sehr geringen Antigenmengen erzielt werden kann, aber auch mit wesentlich höheren Antigenmengen in vergleichbarer Weise erzielt werden kann. Es ist aber naturgemäß wünschenswert, grundsätzlich mit möglichst niedrigen Dosierungen zu arbeiten, insbesondere im Hinblick auf Nebenwirkungen, Materialkosten etc., was bei einer Vakzinierung zum Tragen kommt.

Eine Vakzinierung im Sinne der vorliegenden Erfindung kann grundsätzlich sowohl im therapeutischen Sinne als auch im prophylaktischen Sinne (wie bei allen antimikrobiellen Impfstoffen) durchgeführt werden. Das heißt, die erfindungsgemäße Vakzinierung gegen Krebs kann sowohl als therapeutische als auch als prophylaktische Anwendung verstanden werden. Somit kann durch die Vakzinierung von nicht an Krebs erkrankten Personen mit geeigneten Antikörpern gegebenenfalls ein vorbeugender Schutz gegen den Ausbruch einer Krebserkrankung erzielt werden. Für eine solche prophylaktische Vakzinierung kommen insbesondere, wenn auch nicht ausschließlich, Personen in Frage, die ein erhöhtes Risiko aufweisen, eine Krebserkrankung zu entwickeln.

Die erfindungsgemäße Verwendung unterscheidet sich substantiell von den beiden zuvor genannten, bisher bekannten prinzipiellen therapeutischen Anwendungsmöglichkeiten von Antikörpern zur Behandlung von Krebs und erlaubt eine unerwartet wirksame Behandlung.

Die Bindungsregion eines Antikörpers gegen ein TAA kann nach dem "Schlüssel-Schloß-Prinzip" ein strukturell komplementäres Abbild des Bindungs-Epitopes des jeweiligen TAA's darstellen. Das bedeutet, daß ein solcher Antikörper in seinem Idiotyp eine Strukturinformation des Epitopes des TAA trägt, gegen das er gerichtet ist. Wird daher ein Krebspatient mit einem geeigneten, immunogenen Antikörper gegen ein TAA geimpft (also z.B. mit einem murinen MAK gegen ein TAA), werden dagegen im Patienten Antikörper gebildet, die zum Teil gegen den Idiotyp des als Vakzine eingesetzten Antikörpers gerichtet sind und nach dem "Schlüssel-Schloß-Prinzip" das Epitop des TAA strukturell nachahmen können.

Daraus folgt, daß durch eine solche Impfung im Krebspatienten gewissermaßen lösliche Varianten des Epitopes des TAA generiert werden, die als aktiv induzierte autologe Antikörper für lange Zeiträume wirken können und deren Titer in geeigneten Abständen durch Booster-Impfungen wieder aufgefrischt werden können.

Das gattungsgemäße menschliche zelluläre Membranantigen ist eine Struktur, die eine Rolle spielt bei Adhäsionsvorgängen. Adhäsionsvorgänge sind dabei vorzugsweise Zell-Zell-Interaktionen, wobei Liganden bzw. Rezeptoren auf der Zelloberfläche involviert sind. Adhäsionsmoleküle sind demnach der Zell-Zett-Interaktion dienende Liganden bzw. Rezeptoren auf der Zelloberfläche. Eine Untergruppe von solchen Adhäsionsmolekülen sind die Selbstadhäsionsmoleküle. Diese haben die Eigenschaft, an sich selbst binden zu können.

Die physiologische Wirkung einer durch Impfung mit einem Antikörper gegen ein TAA induzierten Immunantwort hängt naturgemäß von der Funktion des jeweiligen TAA ab. Hat das TAA beispielsweise eine Funktion als Rezeptor für die Adhäsion von Turnorzellen, insbesondere an einen Liganden auf Endothelzellen des Gefäßsystems (eine solche Eigenschaft ist wichtig für die Fähigkeit von disseminierten Krebszellen, aus dem Gefäßsystem auszutreten und sich in Gewebe festzusetzen, um dort eine Metastase auszubilden), dann wird diese Adhäsionsfähigkeit durch Vakzinierung mit einem geeignetem Antikörper gegen dieses TAA herabgesetzt, weil im Kreislauf und im Gewebe permanent induzierte Antikörper vorhanden sind, die die Interaktion des TAA mit seinem Liganden kompetitieren, da sie das TAA in löslicher Form nachahmen.

Allgemein ausgedrückt, kann nach den obenstehenden Ausführungen durch Vakzinierung mit geeigneten Antikörpern gegen TAA, die eine Funktion für die Malignität von Tumorzellen haben, erreicht werden, daß die induzierte Immunantwort mit der Funktion des TAA in dessen Wechselwirkung mit seinem Liganden interferiert und diese erschwert oder verhindert. Das bedeutet, daß Krebszellen für den malignen Phänotyp wichtige Eigenschaften nicht oder nicht ausreichend ausüben können, wodurch die Entwicklung der Erkrankung verlangsamt oder gestoppt werden kann und insbesonders in frühen Stadien die Ausbildung von Metastasen unterdrückt werden kann.

Das gattungsgemäße Membranantigen ist zur Selbstadhäsion fähig, d.h. bestimmte Epitope des Antigens sind für die homophile Bindung mit einem gleichen Antigen auf einer anderen Zelle verantwortlich. Beispiele für solche Antigene sind unter anderem N-CAM (Neuronal Cellular Adhesion Molecule), CEA (Carcino Embryonic Antigen) und Ep-CAM (Epithelial Cell Adhesion Molecule). Antikörper, die gegen Epitope von Selbstadhäsionsantigenen gerichtet sind, welche in diese Funktion involviert sind, können nach den obenstehenden Ausführungen eine komplementäre Strukturinformation eines solchen Epitopes tragen. Daher kann, entsprechend den obenstehenden Ausführungen, durch Vakzinierung mit solchen Antikörpern die Ausbildung von Antikörpern induziert werden, die die Eigenschaften dieser Selbstadhäsion in der Bindungsreaktion tragen. Das bedeutet, daß solche induzierten Antikörper wiederum an

EP 1 230 932 B1

dem Selbstadhäsionsantigen binden können, da in einem solchen Fall Rezeptor und Ligand identisch sind. Damit kann durch Impfung von Krebspatienten mit geeigneten Antikörpern gegen Selbstadhäsionsantigene eine Immunantwort induziert werden, die wiederum direkt an Tumorzellen bindet und dadurch vielfältige therapeutische Wirkungen auslöst. Einerseits wird die für maligne Zellen wichtige Fähigkeit der Selbstadhäsion blockiert und andererseits können durch die Bindung der induzierten Antikörper an die Krebszellen humane Effektorfunktionen wie Komplement-abhängige Lyse und/oder Lyse durch Aktivierung von zytotoxischen Effektorzellen ausgelöst werden, die zur Zerstörung der Krebszellen führen.

Durch alle oben genannten Mechanismen und Wirkungen kann die Vakzinierung von Krebspatienten mit geeigneten Antikörpern gegen TAA die Ausbildung neuer Metastasen unterdrücken und die Disseminierung der Erkrankung zumindestens verlangsamen. In frühen Krankheitsstadien, zum Beispiel nach erfolgreicher Operation eines Primärtumors (adjuvantes Stadium), werden durch solche Impfungen restliche, disseminierte Tumorzellen daran gehindert, sich als neue Metastasen zu etablieren. Dadurch kann die rückfaltsfreie Lebensspanne und damit auch die Gesamtüberlebenszeit solcher Patienten verlängert werden. Durch solche Impfungen und in geeigneten Abständen durchgeführte Auffrischungsimpfungen kann gegebenenfalls ein lebenslanger Schutz vor der Ausbildung von Metastasen erreicht werden. Von besonderem Interesse sind Vakzinierungen von Krebspatienten mit geeigneten Antikörpern gegen ein Selbstadhäsions-TAA, da in diesen Fällen, wie oben beschrieben, durch einen zusätzlichen direkten Angriff der induzierten Immunantwort auf Tumorzellen eine verstärkte therapeutische Wirkung möglich ist

Das gemäß der erfindungsgemäßen Verwendung hergestellte Arzneimittel enthält neben dem Antikörper mindestens ein bei der Formulierung von Vakzinen übliches Adjuvans. Durch solche Adjuvantien kann die Immunantwort verstärkt werden. Als Beispiele für Adjuvantien, jedoch nicht auf diese eingeschränkt, seien erwähnt: Aluminiumhydroxid (Alu-Gel), Derivate von Lipopolysaccharid, Bacillus Calmette Guerin (BCG), Liposomenbereitungen, Formulierungen mit zusätzlichen Antigenen, gegen die das Immunsystem bereits eine starke Immunantwort gemacht hat, wie z.B. Tetanus Toxoid, Pseudomonas Exotoxin, oder Bestandteile von Influenza-Viren, gegebenenfalls in einer Liposomenbereitung, biologische Adjuvantien wie Granulozyten-Makrophagen stimulierender Faktor (GM-CSF), Interleukin 2 (IL-2) oder Gamma Interferon (IFN$\gamma$).

Das gemäß der erfindungsgemäßen Verwendung hergestellte Arzneimittel wird berietgestellt für eine Verabreichung zur Impfung in einer Dosierung von 0,01 bis 4 mg Antikörper, vorzugsweise von 0,5 mg.

Die Impfung kann durch einmalige Applikation der oben angegebenen Dosierung erfolgen. Vorzugsweise erfolgt sie jedoch durch wiederholte Verabreichungen. Die Zahl der Wiederholungen liegt dabei im Bereich von 1 bis 12 pro Jahr, besonders bevorzugt im Bereich von 4 bis 8 pro Jahr. Die Dosierung kann dabei gleichbleiben oder abnehmen.

Auffrischungsimpfungen können dabei in regelmäßigen Abständen, prinzipiell lebenslang, durchgeführt werden. Geeignete Intervalle liegen hierbei im Bereich von etwa 6 bis zu 24 Monaten und können durch Verfolgung der Titer der induzierten Antikörper festgelegt werden (eine Auffrischung sollte erfolgen, sobald die Titer der induzierten Antikörper deutlich abgefallen sind).

Die Verabreichung des Antikörpers kann nach dem Fachmann bekannten Methoden erfolgen. Vorzugsweise ist das Arzneimittel enthaltend den Antikörper geeignet für eine subcutane, intradermale oder intramuskuläre Verabreichung.

Einige wesentliche Vorteile der erfindungsgemäßen Vakzine gegenüber der passiven Anfikörper-Immuntherapie sind wie folgt zusammengefaßt:

**Antikörper gegen TAA zur passiven Immuntherapie von Krebs:**

- Hohe Dosis ($\geq$ 100 mg / intravenöse Infusion)
- Kurze Wirkung durch Elimination des Wirkstoffes
- Xenogene Antikörper unerwünscht wegen Immunogenität
- Therapiedauer insbesondere bei xenogenen Antikörpern wegen der Induktion einer Immunantwort und der dadurch entstehenden Gefahr von anaphylaktischen Reaktionen bei wiederholter Anwendung limitiert

**Antikörper gegen TAA zur prophylaktischen und/oder therapeutischen Vakzinierung gegen Krebs:**

- Niedrige Dosis (< 1 mg/Impfung; subcutane, intradermale oder intramuskuläre Injektion)
- Lang andauernde Wirkung der direkt induzierten Immunantwort
- Xenogene Antikörper erwünscht, da die Wirkung auf Immunogenität beruht

• Behandlungsdauer unlimitiert (Auffrischungsimpfungen immer wieder möglich)

Im folgenden werden Experimente beschrieben, die belegen, daß die Vakzinierung mit einem bestimmten murinen MAK (HE2), der gegen das Selbstadhäsions-TAA Ep-CAM gerichtet ist, oder die Vakzinierung mit seinem F (ab)'$_2$-Fragment, direkt zur Induktion von Antikörpern führt, die selektiv auf menschlichen Tumorzellen binden, die dieses Antigen exprimieren. Damit wird beispielshaft, aber ohne jede Einschränkung gezeigt, daß durch die Vakzinierung mit geeigneten Antikörpern gegen ein Selbstadhäsions-TAA, oder deren Derivaten, die zumindestens den Idiotyp des Ausgangsantikörpers enthalten, eine Immunantwort induziert wird, die therapeutische Wirksamkeit bei Krebserkrankungen haben kann.

Der murine monoclonale Antikörper HE2 wurde dafür nach an sich beschriebenen Standardverfahren der Hybridomtechnologie generiert (siehe z.B. H. Zola. Monoclonal Antibodies: A Manual of Techniques. CRC Press, Inc. ISBN 0-8493-6476-0; 1988) Balb/c-Mäuse wurden mit menschlichen Kolorektal-Krebszellen nach Standardprotokollen immunisiert. Die Milzzellen wurden mit der Maus-Myelomlinie P3X63Ag8 fusioniert und Hybridome selektioniert, die Antikörper produzieren, die selektiv an humane kolorektale Krebszellen, aber nicht an . Melanomzellen binden. Letztlich wurde ein Hybridom ausgewählt, das einen IgG2a/kappa Antikörper sezerniert. Dieser Antikörper (HE2) bindet an Ep-CAM, wie z.B. durch Westernblotanalyse mit Membranpräparationen von KATO III Magenkrebszellen im Vergleich mit einem bekannten anti-Ep-CAM Antikörper (KS1-4) gezeigt werden kann.

Die Aminosäuresequenzen der variablen Regionen des MAK HE2 sind wie folgt: Schwere Kette:

QVQLQQSGAELVRPGTSVKVSCKASGYAFTNYLIEWVKQRPGQGLEWIGVINPG

SGGTNYNEKFKGKATLTADKSSSTAYMQLSSLTSDDSAVYFCARDGPWFAYWG

QGTLVTVSA (SEQ ID NO: 1)

Leichte Kette:

NIVMTQSPKSMSMSVGERVTLTCKASENVVTYVSWYQQKPEQSPKLLIYGASNR

YTGVPDRFTGSGSATDFTLTISSVQAEDLADYHCGQGYSYPYTFGGGTKLEIK

(SEQ ID NO. 2)

Die Figuren zeigen:

**Figur 1**      zeigt die Inhibierung *in vitro* der Selbstadhäsion der kleinzelligen Lungenkrebslinie SW2 durch den MAK HE2.

**Figur 2**      zeigt die Selbstadhäsion *in vitro* der human kleinzeiligen Lungenkrebslinie SW2 ohne Einfluß des MAK HE2, als Kontrolle zu dem in Figur 1 dargestellten Experiment.

**Figur 3**      zeigt die Induktion einer Anfikörperimmunantwort gegen HE2 nach Vakzinierung von Ziegen mit dem F (ab)'2-Fragment von HE2, gemessen in einem ELISA.

**Figur 4**      zeigt die Induktion einer Antikörperimmunantwort gegen Ep-CAM positive human Magenkrebszellen (Kato III) nach Vakzinierung von Ziegen mit dem F(ab)'2-Fragment von HE2, gemessen in einem Zell-ELISA.

**Figur 5**      zeigt das Fehlen einer Anitkörperimmunantwort gegen Ep-CAM negative humane Melanomzellen (WM9) nach Vakzinierung von Ziegen mit dem F(ab)'2-Fragment von HE2, gemessen in einem Zell-ELISA, als Kontrolle zu dem in Figur 4 dargestellten Experiment.

**Figur 6**      zeigt die Bindung einer affinitätsgereinigten Antikörperfraktion aus Serum von Ziegen, die mit dem F(ab) '2-Fragment von HE2 vakziniert wurden, an Ep-CAM positive humane Magenkrebszellen (Kato III), gemessen in einem Zell-ELISA.

**Figur 7**      zeigt das Fehlen der Bindung einer affinitätsgereinigten Antikörperfraktion aus Serum von Ziegen, die mit dem F(ab)'2-Fragment von HE2 vakziniert wurden, an Ep-CAM negative humane Melanomzellen (WM9), gemessen in einem Zell-ELISA, als Kontrolle zu dem in Figur 6 dargestellten Experiment.

**Figur 8**      zeigt die Induktion einer Antikörperimmunantwort gegen HE2 nach Vakzinierung von Rhesusaffen mit 0,5 mg an Aluminiumhydroxid adsorbiertem HE2, gemessen in einem ELISA.

**Figur 9**      zeigt die Induktion einer Antikörperimmunantwort gegen Ep-CAM positive humane Magenkrebszellen (Kato III) nach Vakzinierung von Rhesusaffen mit 0,5 mg an Aluminiumhydroxid adsorbiertem HE2, gemessen in einem Zell-ELISA.

**Figur 10** zeigt die im Rahmen einer Rhesusaffen-Toxizitätsstudie mit HE2 gefundene Induktion einer Antikörperimmunantwort gegen HE2 nach Vakzinierung einer Gruppe von Rhesusaffen mit 0,5 mg an AlL'miniumhydroxid adsorbiertem HE2, sowie das Fehlen einer Immunantwort gegen HE2 nach Behandlung einer anderen Gruppe von Rhesusaffen mit einer Aluminiumhydroxidformulierung ohne Antigen (Plazebo), gemessen in einem ELISA.

**Figur 11** zeigt beispielshaft die im Rahmen der Rhesusaffen-Toxizitätsstudie mit HE2 gefundene Induktion einer Antikörperimmunantwort gegen Ep-CAM positive humane Magenkrebszellen (Kato III), gemessen in einem Zell-ELISA

**Figur 12** zeigt die Induktion einer Antikörperimmunantwort gegen Ep-CAM positive humane Magenkrebszellen (Kato III) nach wiederholter Vakzinierung eines Darmkrebspatienten mit je 0,5 mg an Aluminiumhydroxid adsorbiertem HE2, gemessen in einem Zell-ELISA.

**Figur 13** zeigt die Induktion einer Serum-Zytotoxizität gegen Ep-CAM positive humane Magenkrebszellen (Kato III) nach wiederholter Vakzinierung eines Darmkrebspatienten mit je 0,5 mg an Aluminiumhydroxid adsorbiertem HE2, gemessen in einem Zell-Lyse-Experiment.

Die folgenden Beispiele sollen die vorliegende Erfindung weiter erläutern, aber nicht einschränken:

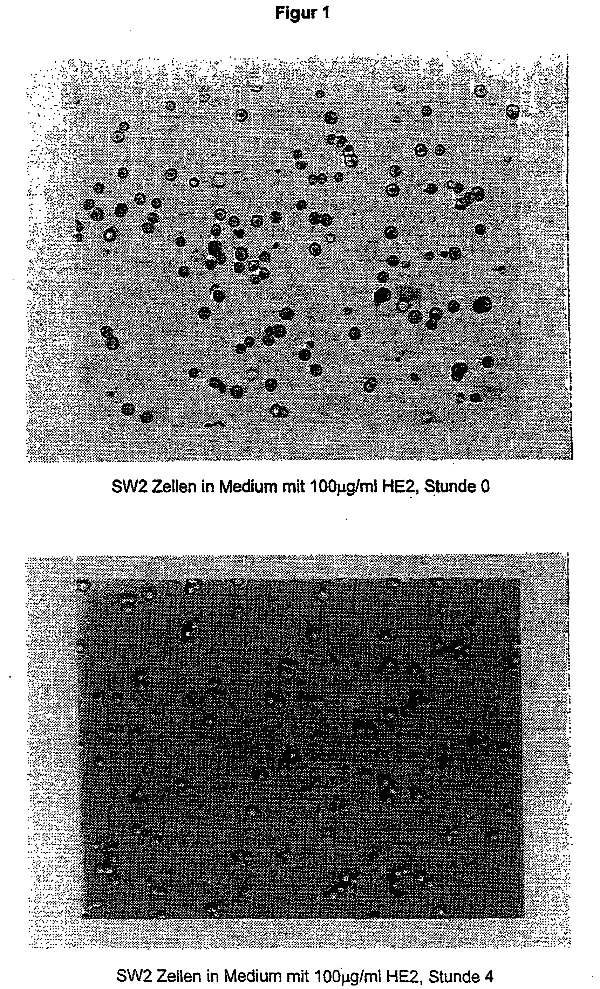

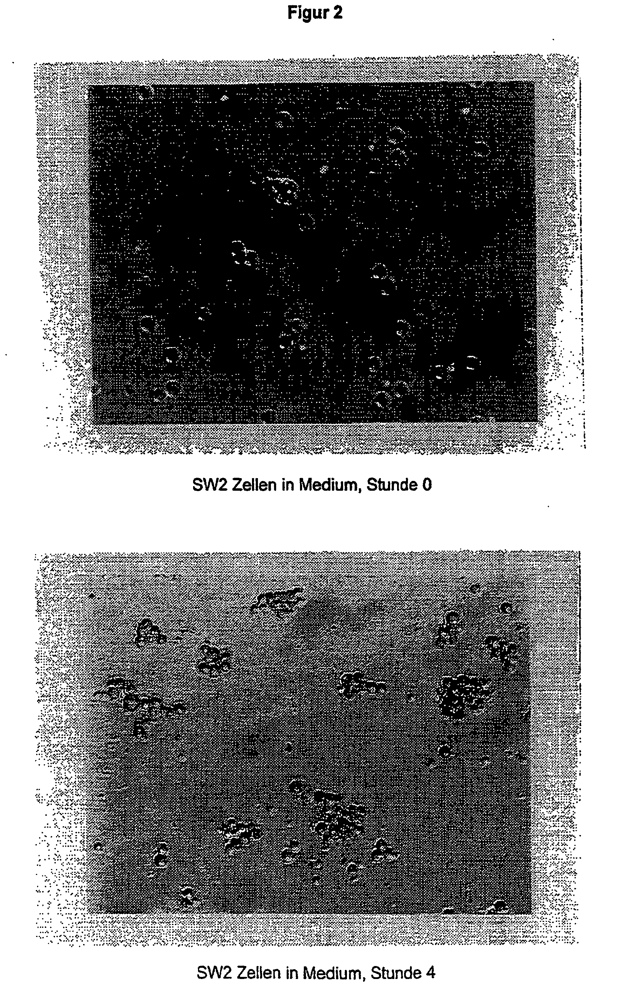

Um zu zeigen, daß der murine MAK HE2 an einem Epitop des Selbstadhäsionsantigens Ep-CAM bindet, das in die homophile Bindung involviert ist, wurde der Einfluß von HE2 auf die Selbstadhäsionsfähigkeit der humanen Zelllinie SW2 untersucht. Diese kleinzellige Lungenkarzinomlinie neigt dazu, sich in Kultur in vitro nach vorhergehender Vereinzelung innerhalb von einigen Stunden zu Zellklustern zusammenzuschließen. Die experimentelle Beschreibung befindet sich in Beispiel 1. Wie aus den Figuren 1 und 2 ersichtlich ist, wird durch die Zugabe von HE2 die Ausbildung von Zetlklustern weitestgehend verhindert. Damit wird bewiesen, daß HE2 an ein Epitop von Ep-CAM bindet, das in die homophile Bindung dieses Membranproteines involviert ist.

Um die direkte humorale Immunantwort auf die Vakzinierung mit dem F(ab)'$_2$-Fragment des murinen MAK HE2 untersuchen zu können, wurden Ziegen mit diesem Fragment immunisiert. Das Fragment wurde nach an sich bekannten und beschriebenen Methoden durch Spaltung von HE2 mit Pepsin hergestellt und gereinigt. Die Immunisierung der Ziegen ist in Beispiel 2 beschrieben.

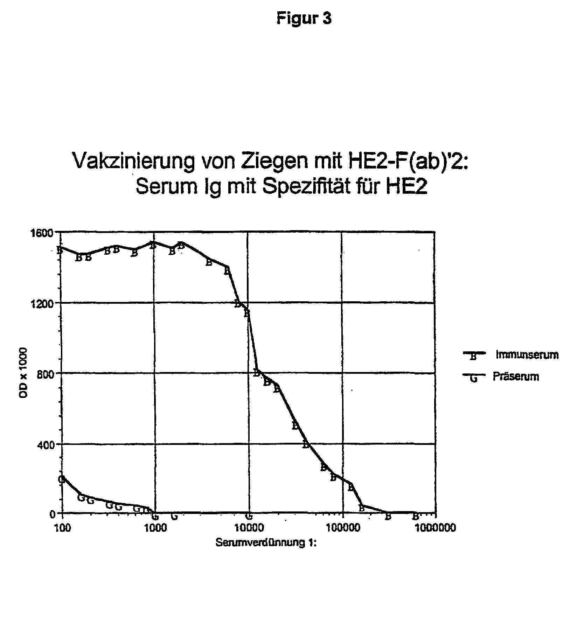

Zunächst wurde das gewonnene und gepoolte Ziegen-Immunserum im Vergleich zu einem Prä-Serum auf Immunglobuline untersucht, die gegen den MAK HE2 gerichtet sind, um die Total-Immunantwort der vakzinierten Ziegen zu bestimmen. Diese Untersuchung wurde mit Hilfe eines ELISA Tests durchgeführt, die experimentelle Beschreibung befindet sich in Beispiel 3. Das Ergebnis dieses Experimentes ist in Figur 3 dargestellt: Die Ziegen haben durch die Vakzinierungen mit dem F(ab)'$_2$-Fragment des MAK HE2 eine starke Immunantwort dagegen aufgebaut, während in einem Prä-Serum keine Antikörper gegen HE2 gefunden werden.

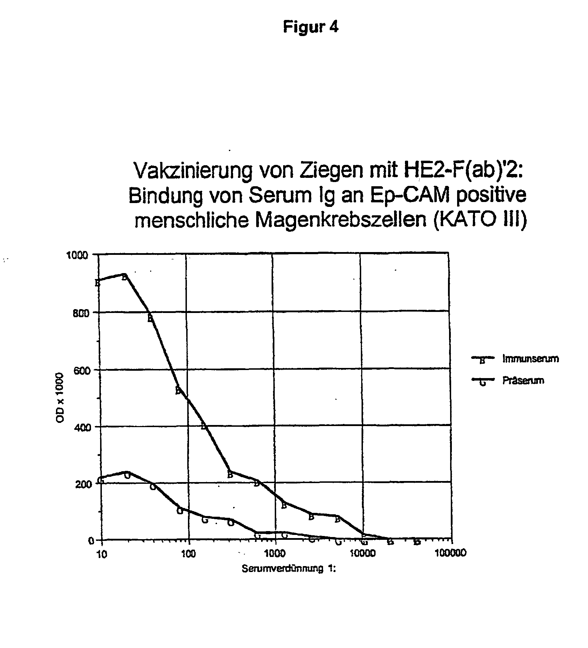

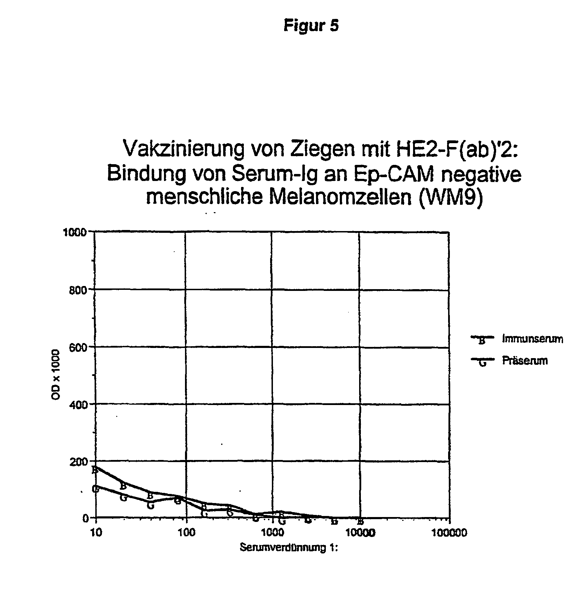

Als nächstes wurde untersucht, ob sich in dem Ziegen-Immunserum Immunglobuline nachweisen lassen, die an humane Krebszellen binden, die das TAA exprimieren, gegen das der MAK HE2 gerichtet ist (Ep-CAM). Dafür wurde die KATO III Magenkrebszellinie herangezogen. Als Kontrolle wurde auch die Bindung an eine humane Zellinie getestet, die Ep-CAM nicht exprimiert (WM9 Melanomzellen). Diese Untersuchungen wurden mit Hilfe von Zell-ELISA Tests durchgeführt, die experimentelle Beschreibung befindet sich in Beispiel 4. Das Ergebnis dieser Experimente ist in den Figuren 4 und 5 dargestellt: Das Ziegen-Immunserum enthält Immunglobuline, die stark auf den Ep-CAM positiven KATO-Zellen binden, während keine Bindung auf den Ep-CAM negativen WM9-Zellen nachgewiesen wird. Im Prä-Serum befinden sich keine Antikörper, die an diese Zellen binden. Dieses sehr überraschende Ergebnis zeigt, daß durch Vakzinierung mit HE2-F(ab)'$_2$ Fragment entstandene Antikörper tatsächlich in der Lage sind, selbst wieder an Zellen zu binden, die das TAA exprimieren, das HE2 erkennt. Danach konnte die Funktion des TAA zur Selbst-Adhäsion auf diese durch die Impfung mit HE2 entstandenen Antikörper übertragen werden, wie zuvor ausführlich erläutert wurde.

Um zu beweisen, daß die in den Ziegen durch Impfung mit dem F(ab)'$_2$-Fragment von HE2 gegen den Idiotyp dieses MAK entstandenen Antikörper tatsächlich diejenigen sind, die an die KATO-Zellen binden, wurde der anti-idiotypische Anteil dieser induzierten Antikörper mit Hilfe einer Sequenz von Immunaffinitätschromatographien spezifisch aus dem Ziegen-Immunserum gereinigt, wie grundsätzlich beschrieben (Proc. Nati. Acad. Sci. USA 81 (1984), 216). Die Sequenz der Reinigungsschritte ist in Beispiel 5 noch einmal zusammengefaßt.

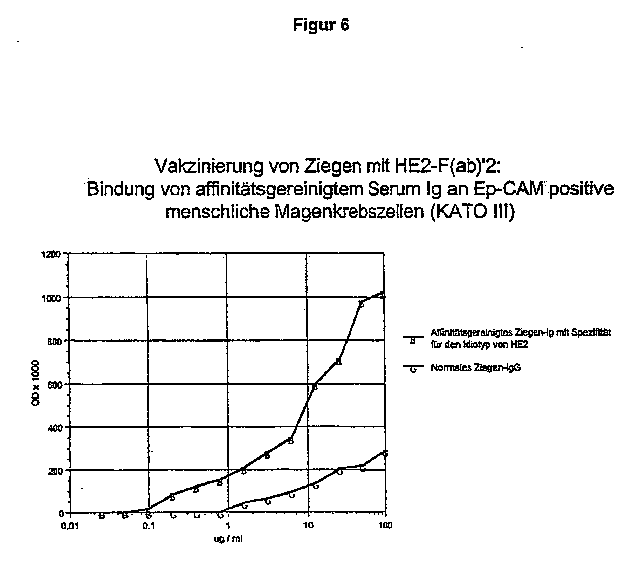

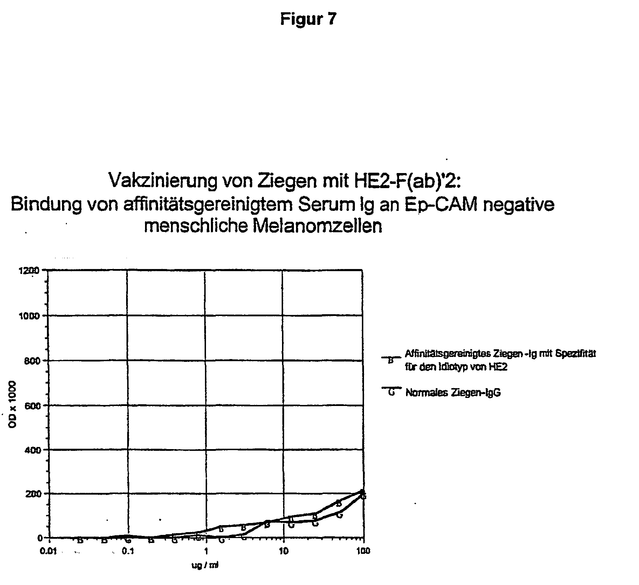

Diese affinitätsgereinigten Ziegenantikörper wurden wieder auf ihre Bindung an die Ep-CAM positiven KATO-Zellen sowie an die Ep-CAM negativen WM9-Zellen untersucht, die experimentelle Beschreibung befindet sich in Beispiel 6. Das Ergebnis dieser Experimente ist in den Figuren 6 und 7 dargestellt: Das Ziegen-IgG, das gegen den Idiotyp von HE2 gerichtet ist, bindet stark an die Ep-CAM positiven KATO-Zellen, während unspezifisches Ziegen-IgG kaum bindet. Die Bindung des affinitätsgereinigten spezifischen Ziegen IgG an die Ep-CAM negativen WM9-Zellen unterscheidet sich hingegen nicht von der des unspezifschen Ziegen-IgG. Damit ist bewiesen, daß die Fraktion der Antikörper, die durch Vakzinierung mit dem F(ab)'$_2$-Fragment von HE2 gegen den Idiotyp dieses Antikörpers direkt entstanden ist, diejenigen Antikörper enthält, die an den Krebszellen binden, die das TAA exprimieren, das HE2 erkennt. Durch dieses Experiment ist auch schlüssig gezeigt, daß die durch Vakzinierung mit HE2 induzierten Antikörper gegen Ep-CAM positive Zellen nicht durch eine doppelte autologe idiotypische Netzwerkkaskade entstanden sind, wie in einigen Publikationen postuliert worden ist (siehe z.B.: Cancer Immunol Immunother 42 (1996), 81-87,). Solche anti-anti-idiotypischen Antikörper (Ab3) könnten nämlich in keiner Weise durch Affinitätschromatographie an einer Ab1 Säule (=HE2) gereinigt werden, da sie entsprechend dem idiotypischen Netzwerk nicht an Ab1, sondern nur an Ab2 binden können.

Aufgrund der oben dargestellten Ergebnisse der Immunisierung von Ziegen mit dem F(ab)'$_2$-Fragment von HE2 wurden Vakzinierungsstudien auch mit Rhesusaffen durchgeführt, um die immunologischen Resultate in einer dem Menschen nahestehenden Spezies zu bestätigen. Für diese Experimente wurde der gesamte murine MAK HE2 als Immunogen verwendet. Es wurde angenommen, daß der murine Fc Teil als großes Fremdprotein die Immunantwort auch gegen den Idiotyp verstärkt (Carrier Effekt). Um mögliche lokale Nebenwirkungen zu vermeiden, wurde Aluminiumhydroxid als mildes Adjuvans verwendet. Die Herstellung der Formulierung für diese Impfversuche wird in Beispiel 7 beschrieben.

Vier Rhesusaffen wurde die in Beispiel 7 beschriebene Formulierung subkutan in den Rücken injiziert (0,5 mg HE2 = 0,5 ml pro Impfung, 2 mal verabreicht im Abstand von 4 Wochen). Zu mehreren Zeitpunkten wurde Blut zur Serumgewinnung abgenommen.

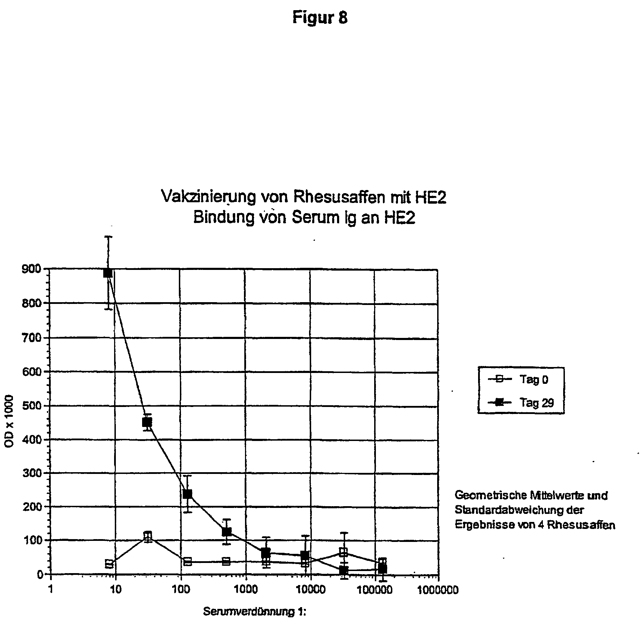

Zunächst wurde die Immunantwort gegen HE2 in einem ELISA gemessen. Die experimentelle Beschreibung befindet sich in Beispiel 8. Wie in Figur 8 gezeigt wird, werden bereits am Tag 29 deutliche Titer von Antikörpern gegen HE2 gemessen.

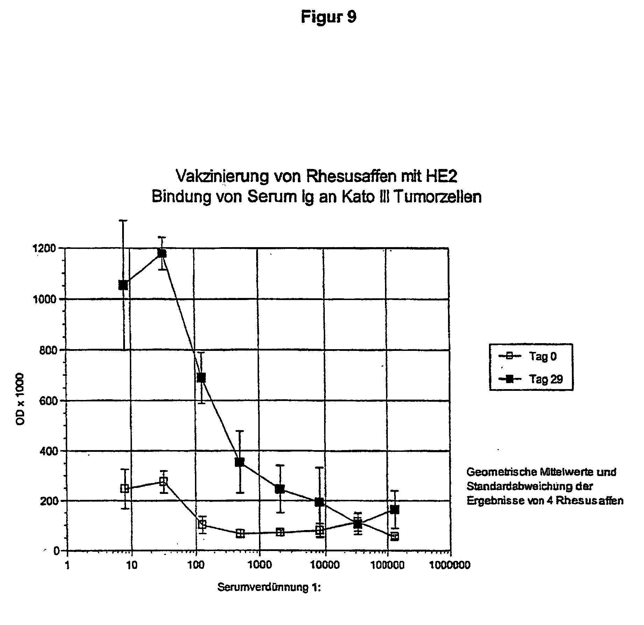

Weiters wurde untersucht, ob durch die Impfungen Antikörper induziert werden, die an KATO III Zellen binden. Für diese Tests wurde ein Zell-ELISA eingesetzt, die experimentelle Beschreibung befindet sich in Beispiel 9. Wie in Figur 9 gezeigt wird, sind bereits am Tag 29 in allen Tieren Antikörper induziert, die auf Ep-CAM positiven Kato III Tumorzellen binden.

In weiterer Folge wurden im Rahmen einer Toxizitätsstudie mit Rhesusaffen vier Tiere mit an Aluminiumhydroxid adsorbierten HE2 geimpft. Vier weitere Rhesusaffen erhielten als Plazebo nur Aluminiumhydroxid. Die Herstellung der Formulierungen ist in den Beispielen 10 und 11 beschrieben. Den Rhesusaffen wurde insgeamt 4 mal je 0,5 ml der jeweiligen Formulierung (Wirkstoff oder Plazebo) subkutan in den Rücken injiziert (Tage 1, 15, 29 und 57). Vor Beginn der Studie und zu verschiedenen Zeiten während der Behandlungen wurde Blut für Serumgewinnung abgenommen.

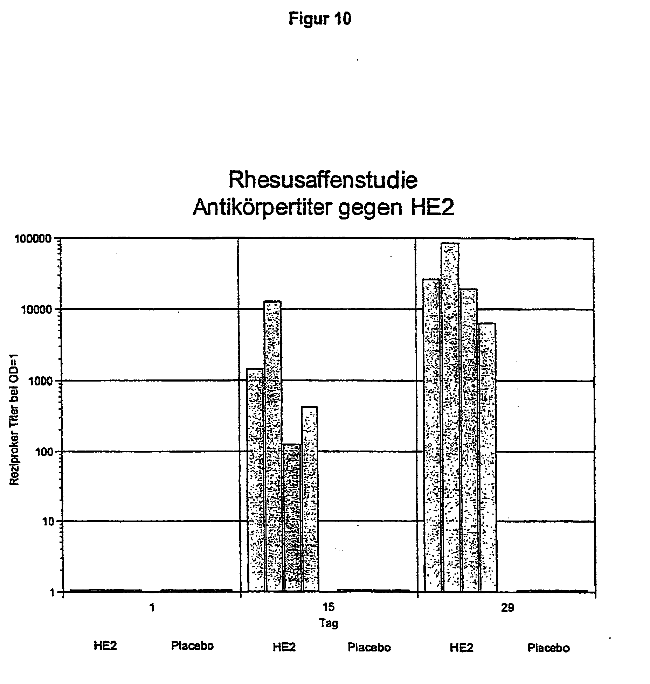

Zunächst wurde wieder die Immunantwort gegen HE2 in einem ELISA gemessen. Die experimentelle Beschreibung befindet sich in Beispiel 8. Wie in Figur 10 gezeigt wird, haben alle vier Rhesusaffen aus der HE2-Gruppe bereits nach einer Impfung eine deutliche humorale Immunantwort gegen HE2 aufgebaut, die durch die zweite Impfung noch verstärkt wird, während die Rhesusaffen aus der Plazebogruppe keinerlei Erhöhung der Titer von Antikörpern gegen HE2 aufweisen.

Diese Befunde wurden weiters durch Immunaffinitätsreinigungen der Seren Tag 43 der Rhesusaffen der HE2-Gruppe bestätigt. Die experimentelle Beschreibung befindet sich in Beispiel 12. Wie in folgender Tabelle gezeigt wird, haben alle vier Affen am Tag 43 in ihrem Serum eine starke IgG-Immunantwort gegen HE2 aufgebaut (sekundäre Immunantwort), der IgM-Anteil ist hingegen mit dem der Präseren vergleichbar.

| Affe | Tag | $\mu$g / ml IgM gegen | $\mu$g / ml IgG gegen |
|---|---|---|---|
| 9206m | -14 | 7,7 | 2,8 |
| | 43 | 16,3 | 135,2 |
| 9599m | -14 | 17,9 | 2,5 |
| | 43 | 25,4 | 449,3 |
| 8415f | -14 | 16,0 | 3,2 |
| | 43 | 22,5 | 159,9 |
| 9139f | -14 | 5,3 | 5,0 |
| | 43 | 10,3 | 69,8 |

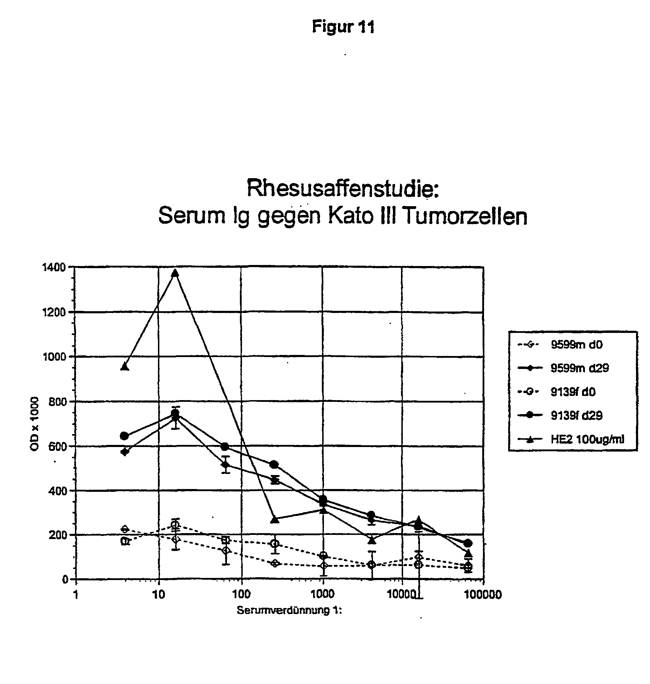

Es wurde auch die Induktion von Antikörpern gegen Ep-CAM positive Kato III Zellen untersucht. Für diese Tests wurde wieder ein Zell-ELISA eingesetzt, die experimentelle Beschreibung befindet sich in Beispiel 9. Wie in Figur 11 beispielshaft gezeigt wird, haben Rhesusaffen aus der HE2-Gruppe bereits am Tag 29 Antikörper gegen Kato III Zellen entwickelt.

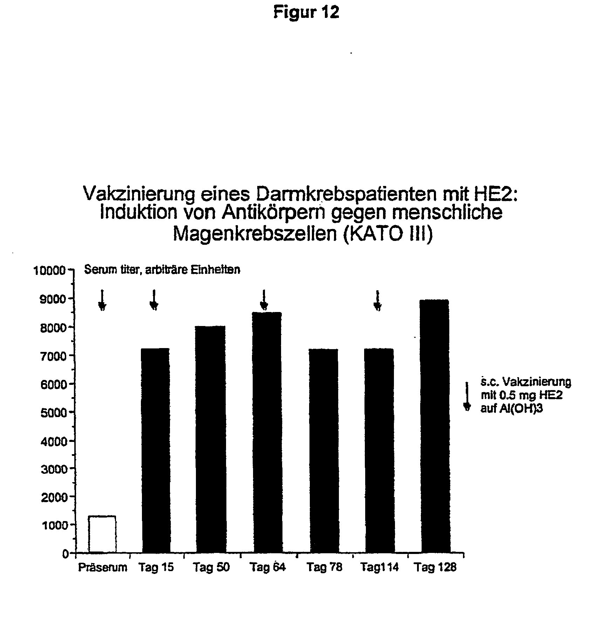

In weiterer Folge und aufgrund der oben dargestellten Ergebnisse der Vakzinierung von Ziegen und Rhesusaffen wurde ein Darmkrebspatient mit Metastasen (Dukes D) im Sinne einer Einzelbeobachtung mit dem MAK HE2, adsorbiert an Aluminiumhydroxid, geimpft. Die Herstellung der dafür verwendeten Formulierung ist in Beispiel 7 beschrieben. Diesem Patienten wurden je 0,5 ml dieser Formulierung (entspricht 0,5 mg HE2) insgesamt 4 mal subkutan in die oberen Extremitäten injiziert (Tag 1, 50, 78, 114). Vor jeder Impfung und am Tag 128 wurde Blut zur Serumgewinnung abgenommen. Es wurde zunächst untersucht, ob durch die Impfungen Antikörper induziert werden, die an KATO III-Zellen binden. Für diese Tests wurde wieder der Zell-ELISA eingesetzt, die experimentelle Beschreibung befindet sich in Beispiel 9. Die Ergebnisse dieser Experimente sind in Figur 12 gezeigt. Durch die Impfungen werden in diesem Krebspatienten offensichtlich hohe Titer von Antikörpern induziert, die an KATO III Zellen binden.

Weiters wurde untersucht, ob die durch die Vakzinierung mit HE2 induzierten Antikörper ex vivo einen zytotoxischen Effekt gegen KATO III Krebszellen vermitteln. Zu diesem Zweck wurden KATO III Zellen mit Prä- und Immunseren dieses Krebspatienten inkubiert, um eine duch die induzierten Antikörper mediierte Komplement abhängige Lyse zu zeigen. Die experimentelle Beschreibung befindet sich in Beispiel 13.

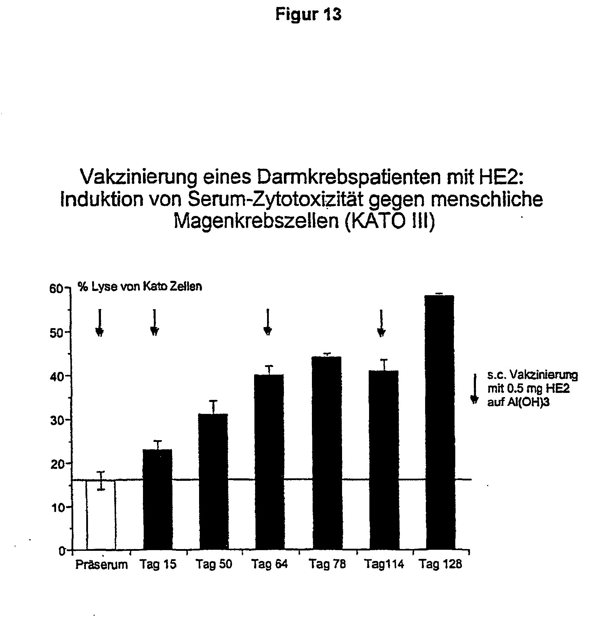

Die Ergebnisse sind in Figur 13 dargestellt. Die durch die Impfung mit HE2 induzierten Antikörper sind offensichtlich in autologem Patientenserum in der Lage, mittels Komplement-abhängiger Lyse Ep-CAM positive KATO III-Zellen zu zerstören.

Auf Grund der oben beschriebenen Experimente ist beispielhaft gezeigt, daß die Vakzinierung mit geeigneten Antikörpern gegen ein Selbstadhäsions-TAA wie z.B. Ep-CAM, oder deren Derivaten mit gleichem Idiotyp wie die jeweiligen Ausgangsantikörper, eine humorale Immunantwort auslöst, die selektiv auf Tumorzellen bindet, die dieses Selbstadhäsions-TAA exprimieren. Die induzierten Antikörper können ein zytotoxisches Potential gegen solche Tumorzellen aufweisen. Eine Vakzinierung mit einem solchen Antikörper kann damit zu einer therapeutischen Wirkung bei Krebserkrankungen führen.

**Beispiele**

Verwendete Materialien:

| | |
|---|---|
| Mikrotiterplatten: | Immuno Plate F96 MaxiSorp (Nunc) für ELISA |
| | Cell Culture Cluster (Costar, Cat.Nr. 3598) für Zell-ELISA |
| Zellinien: | SW2: menschliche kleinzellige Lungenkarzinomlinie, Ep-CAM positiv |
| | KATO III: menschliche Magenkrebszellinie, Ep-CAM positiv      (ATCC HTB 103) |
| | WM 9: menschliche Melanomzellinie, Ep-CAM negativ |
| Kopplungspuffen: | 0,1 M NaHCO$_3$ |
| | 0,5 M NaCl |
| | pH-Wert 8,0 |
| Reinigungspuffer A: | PBS def |
| | 0,2 M NaCl |
| | pH-Wert 7,2 |
| Reinigungspuffer B: | 0,1 M Glycin / HCl |
| | 0,2 M NaCl |
| | pH-Wert 2,9 |
| Medium A: | RPMI 1640 + 2 g/l NaHCO$_3$ |
| | 100 U/ml Penicillin G |
| | 100 µg/ml Streptomycinsulfat |
| | 4 mM Glutamin |
| | 10 % foetales Kälberserum (hitzeinaktiviert) |
| Bindungspuffer: | 15 mM Na$_2$CO$_3$ |
| | 35 mM NaHCO$_3$ |
| | 3 mM NaN$_3$ |
| | pH-Wert: 9,6 |
| PBS deficient: | 138 mM NaCl |

1,5 mM KH$_2$PO$_4$
2,7 mM KCl
6,5 mM Na$_2$HPO$_4$
pH-Wert 7,2

| | |
|---|---|
| Fixierlösung: | 0,1 % Glutardialdehyd in physiologischer NaCl-Lösung |
| Waschpuffer A: | 2% NaCl<br>0,2% Triton X-100<br>in PBS deficient |
| Waschpuffer B: | 0,05 % Tween 20 in PBS deficient |
| Blockterungspuffer A: | 5 % foetales Kälberserum (hitzeinaktiviert) in PBS deficient |
| Blockierungspuffer B: | 1 % Rinderserumalbumin<br>0,1 % NaN$_3$<br>in PBS deficient |
| Verdünnungspuffer A: | 2% foetales Kälberserum (hitzeinaktiviert)<br>in PBS deficient |
| Verdünnungspuffer B: | PBS deficient |
| Färbepuffer: | 24,3 mM Zitronensäure<br>51,4 mM Na$_2$HPO$_4$<br>pH-Wert: 5,0 |
| Substrat: | 40 mg o-Phenylendiamin Dihydrochlorid<br>100 ml Färbepuffer<br>20 μl H$_2$O$_2$ (30%) |
| Stopplösung: | 4 N H$_2$SO$_4$ |

**Beispiel 1:**

In vitro kultivierte SW2 Zellen werden abzentrifugiert, das Pellet in Medium A aufgenommen und auf 7x10$^4$ Zellen/ml eingestellt. In den Kammern eines LabTeks werden entweder 0,1 ml PBS def mit 0,3 ml der Zellsuspension, oder 0,1 ml PBS def mit 40μg HE2 und dann mit 0,3 ml der Zellsuspension gemischt (Endkonzentration HE2 100μg/ ml). Unmittelbar bevor die Zellsuspension jeweils als Letztes zupipettiert wird, werden die Zellen mit der Pipette vereinzelt. Gleich nach Zusammenfügen werden die jeweiligen Zellsuspensionen im Umkehrmikroskop fotografiert (Vergrößerung 100-fach). Anschließend werden die Zellsuspensionen 4 Stunden bei 37°C / 5% CO$_2$ kultiviert und dann wieder fotografiert.

**Beispiel 2:**

Zwei Ziegen werden mit je 1,5 mg des F(ab)' 2-Fragmentes in 3 ml PBS deficient zusammen mit 3ml von Freunds Complete Adjuvant (Difco) an multiplen Stellen intradermal vakziniert. Am Tag 8 wird eine erste Booster-Impfung wie an Tag 1 gegeben, aber mit Freunds Incomplete Adjuvans (Difco). Am Tag 29 wird in gleicher Weise eine zweite Booster-Impfung gegeben, bei der aber kein Adjuvans zugesetzt wird. Blut zur Serumgewinnung für die Analyse der entstandenen Immunantwort wird vor Impfbeginn und am Tag 54 abgenommen.

**Beispiel 3:**

100 μl Aliquots des MAK HE2 (Lösung mit 10μg/ml in Bindungspuffer) werden in den Löchern einer Mikrotiterplatte 1 Stunde bei 37°C inkubiert. Nach sechsmaligem Waschen der Platte mit Waschpuffer A werden je μl des Blockierungspuffers A zugesetzt und 30 Minuten bei 37°C inkubiert. Nach Waschen der Platte wie oben beschrieben werden je 100µl Aliquots der zu testenden Ziegenseren in Verdünnungen von 1:100 bis 1:1 000 000 in Verdünnungspuffer A 1 Stunde bei 37°C inkubiert. Nach Waschen der Platte wie oben beschrieben werden je 100pl des Peroxidase-konjugierten Kaninchen anti-Ziegen-Ig Antikörpers (Zymed) in einer Verdünnung von 1:1000 in Verdünnungspuffer A zugesetzt und 30 Minuten bei 37°C inkubiert. Die Platte wird viermal mit Waschpuffer A und zweimal mit Färbepuffer gewaschen. Die Antikörperbindung wird durch Zusatz von je 100µl des spezifischen Substrats nachgewiesen und die Farbreaktion durch Zugabe von je 50µl Stopplösung nach ca. 10 Minuten abgebrochen. Die Auswertung erfolgt durch Messen der optischen Dichte (OD) bei 490nm (Wellenlänge der Referenzmessung ist 620nm).

**Beispiel 4:**

Die Löcher einer Mikrotiterplatte werden mit je 100 µl einer Zellsuspension der zu testenden Zellinie in der Konzentration von $2 \times 10^6$ Zellen/ml in Medium A über Nacht bei +4°C inkubiert. Nach Absaugen des Überstandes wird die Platte mit je µl Fixierlösung pro Loch 5 Minuten bei Raumtemperatur inkubiert. Nach Absaugen des Überstandes werden je 200µl Blockierungspuffer B zupipettiert und die Platte 1 Stunde bei 37°C inkubiert. Nach zweimaligem Waschen mit je 200µl Waschpuffer B werden je 100µl Aliquots der zu testenden Ziegenseren in Verdünnungen von 1:10 bis 1:100 000 in Verdünnungspuffer B 1 Stunde bei 37°C inkubiert. Nach zweimaligem Waschen der Platte mit je 100 µl eiskaltem Waschpuffer B werden je 100µl des Peroxidase- konjugierten Kaninchen anti-Ziegen-Ig Antikörpers (Zymed) in einer Verdünnung von 1:1000 in Verdünnungspuffer A zugesetzt und 45 Minuten bei 37°C inkubiert. Die Platte wird dreimal mit je 100 µl eiskaltem Waschpuffer B gewaschen. Die Antikörperbindung wird durch Zusatz von je 100µl des spezifischen Substrats nachgewiesen und die Farbreaktion durch Zugabe von je 50µl Stopplösung nach ca. 10 Minuten abgebrochen. Die Auswertung erfolgt durch Messen der optischen Dichte (OD) bei 490nm (Wellenlänge der Referenzmessung ist 620nm).

**Beispiel 5:**

Die Reinigung ist grundsätzlich in Proc.Natl.Acad.Sci.USA 81:216, 1984 beschrieben und wird hier wie folgt zusammengefaßt: In einem ersten Schritt wird nach bekannten Methoden an einer DEAE Anionenaustauscher Säule eine Reinigung des im Ziegenserum enthaltenen Gesamt-IgG durchgeführt. Anschließend werden an einer Immunaffinitätssäule (CH-Sepharose 4B, Pharmacia), an die irrelevantes murines IgG2a gekoppelt wurde, diejenigen Ziegenantikörper gebunden, die gegen konstante Regionen des F(ab)' $_2$-Fragmentes von HE2 gerichtet sind, während die Fraktion der anti-idiotypischen Ziegenantikörper nicht an dieser Säule bindet. In einem letzten Schritt wirde daher der Durchbruch dieser Immunaffinitätschromatographie an einer Immunaffinitätssäule (CH-Sepharose 4B, Pharmacia) gebunden, an die HE2 gekoppelt wurde. Die spezifisch an diese Säule gebundene Fraktion wird mit einem Puffer pH 2,8 (0,1 M Glycin/HCl) eluiert und neutralisiert. Die so erhaltene Ziegen IgG Fraktion ist gegen den Idiotyp von HE2 gerichtet

**Beispiel 6**:

Dieser Zell-ELISA wird grundsätzlich gleich ausgeführt wie in Beispiel 4 beschrieben. Statt Serumverdünnungen werden Konzentrationen von 100 µg/ml bis 0,031µg/ml des immunaffinitätsgereinigten Ziegen IgG bzw. von unspezifischem gereinigten Ziegen IgG verwendet.

**Beispiel 7:**

0,83 ml einer Suspension von Alu-Gel (Alu-Gel S von Serva, 2% Suspension, Qualitätsgrad: Adjuvans zur Präparation von Vakzinen) wird unter sterilen Bedingungen rrit 0,5 ml einer Lösung von 10 mg/ml HE2 in PBS pH 5,5 zusammen mit 3,67 ml PBS def. eine Stunde bei Raumtemperatur sanft geschwenkt (Endkonzentration HE2: 1 mg/ml; Alu-Gel S: 0,33 %). Die Suspension wird anschließend in 0,5 ml Aliquots in Durchstichfläschchen steril abgefüllt.

**Beispiel 8:**

Dieser ELISA wird grundsätzlich gleich ausgeführt wie in Beispiel 3 beschrieben, mit der Ausnahme, daß zur Detektion der gebundenen Rhesusaffenantikörper ein Peroxidase konjugierter Ziegen-anti-Human-Ig Antikörper (Zymed) verwendet wird. Mit diesem Reagens lassen sich Rhesusaffenantikörper in gleicher Weise wie Humanantikörper nachweisen, da die Sequenzhomologie der Konstantregionen von Humanantikörpem und Rhesusaffenantikörpern bei ca. 98% liegt.

**Beispiel 9:**

Dieser Zell-ELISA wird grundsätzlich gleich ausgeführt wie in Beispiel 4 beschrieben, mit der Ausnahme, daß zur Detektion der an die Zellen gebundenen Rhesusaffenantikörper (bzw. Humanantikörper) ein Peroxidase konjugierter Ziegen-anti-Human-Ig Antikörper (Zymed) verwendet wird. Zur Detektion des murinen HE2 als Standard wird ein Peroxidase konjugierter Ziegen-anti-Maus-IgG Antikörper (Zymed) eingesetzt.

**Beispiel 10:**

3,5 ml einer Lösung von HE2 (10 mg/ml in PBS def. pH=5,5) werden unter sterilen Bedingungen mit 0,35 ml einer wässrigen Thimerosallösung (10 mg /ml; Sigma) sowie 27,25 ml physiologischer Kochsalzlösung gemischt und 3,9 ml einer Aluminium-hydroxidsuspension (3% in Wasser; Alhydrogel, Superfos Biosector, Dänemark) unter vorsichtigem Schwenken versetzt. Je 0,6 ml der so erhaltenen Suspension werden dann unter sterilen Bedingungen in entpyrogenisierte Glasröhrchen abgefüllt und mit einem Gummistopfen mit Aluminiumkappe verschlossen.

**Beispiel 11:**

Die Plazeboformulierung wird in gleicher Weise hergestellt wie in Beispiel 10 beschrieben, nur daß statt der Antikörperlösung 3,5 ml PBS def pH=5,5 und statt der Thimerosallösung 0,35 ml physiologischer NaCl-Lösung verwendet werden.

**Beispiel 12:**

1 g CH-Sepharose 4B (Pharmacia) werden für 15 Minuten in 30 ml 1 mM HCI suspendiert. Das Gel wird dann mit 1 Liter 1 mM HCI und anschließend mit 200 ml Kopplungspuffer auf einem Sinterglasfilter AG3 gewaschen. 10 mg HE2 (Stammlösung 10 mg/ml) werden gegen ca 0,5 Liter Kopplungspuffer dialysiert. Diese Lösung wird mit der Gelsuspension in einem verschlossenen Gefäß vermischt. Ein Verhältnis von Gel : Puffer von 1 : 2 ergibt eine für die Kopplung geeignete Suspension. Diese Suspension wird 5,5 Stunden bei Raumtemperatur geschüttelt. Anschließend wird der Überschuß des Liganden mit 3 x 30 ml Kopplungspuffer weggewaschen. Überbleibende reaktive Gruppen werden durch einstündige Inkubation bei Raumtemperatur mit 1 M Äthanolamin geblockt. Dann wird das Gel für eine Stunde bei Raumtemperatur mit einem 0,1 M Tris-HCI Puffer pH=8 geschüttelt. Letztlich wird das Gel mit 3 Zyklen von Puffern mit alternierendem pH gewaschen. Jeder Zyklus besteht aus 0,1 M Natriumacetat Puffer pH 4 mit 0,5 M NaCI, und anschließend 0,1 M Tris-HCI Puffer pH 8 mit 0,5 M NaCI. Das Gel wird bei 4°C aufbewahrt.

Die Immunaffinitätsreinigung der gegen HE2 gerichteten Antikörperfraktion aus Serum der Rhesusaffen erfolgt nach folgender Vorschrift: Die Immunaffinitätsreinigung wird auf einem FPLC-System (Pharmacia) durchgeführt. 1 ml des nach obiger Vorschrift erhaltenen Gels wird in eine Pharmacia HR5/5 Säule gefüllt. 0,5 ml Serum werden 1: 10 mit dem Reinigungspuffer A verdünnt. Diese Lösung wird mit 1 ml / Minute über die Säule gepumpt und mit Reinigungspuffer A nachgewaschen, bis die UV-Basislinie des Detektors wieder erreicht ist (280 nm). Gebundene Immunglobuline werden dann mit Reinigungspuffer B eluiert und die Fraktion mit 0,5 M $Na_2HPO_4$ unmittelbar nach Desorption neutralisiert und mit 0,02% $NaN_3$ versetzt 50µl der so gereinigten Antikörperfraktion werden auf einer Größentrennungssäule (SEC, Zorbax 250 GF) analysiert und die IgG- und IgM- Anteile quantifiziert. Als Laufmittel für die SEC wird 220 mM Phosphatpuffer pH 7 + 10% Acetonitril verwendet. Als Standard für die SEC dient Human IgG und Human IgM, die zum Erstellen einer Standard-Eichgerade jeweils in mehreren Konzentrationen chromatographiert werden (Peakfläche vs. Konzentration). Die Berechnung der IgG- und IgM-Konzentrationen in den affinitätsgereinigten Rhesuaffen-Antikörperfraktionen erfolgt mittels Linearregression anhand der Standardkurven. Die Konzentrationen werden als µg / ml eingesetzten Affenserums angegeben.

**Beispiel 13:**

Ein Tag vor Durchführung des Tests werden KATO III Zellen in frisches Medium A transferiert und bei 37°C/ 5% $CO_2$ in einer Zellkulturliasche gehalten. Am nächsten Tag werden die Zellen zunächst mit [51]Chrom markiert. Dabei werden $5x10^6$ Zellen in 800 µl Medium A bei 37°C/5% $CO_2$ mit 100 µci $Na_2{}^{51}CrO_4$ inkubiert, anschließend mit Medium A gewaschen und auf eine Dichte von $2.5x10^5$ Zellen/ml adjustiert. 100 µl Aliquots dieser Zellsuspension werden in Löcher einer Mikrotiterplatte pippetiert. 100 µl Aliquots der zu testenden Patientenseren werden zugegeben und 3 Stunden bei 37°C/5% $CO_2$ inkubiert (die Seren werden bei -80°C aufbewahrt und für diesen Test nur einmal aufgetaut, um die Aktivität des Komplements nicht zu beeinträchtigen). Die Überstände werden mit einer Skatron-Harvesting-Presse geerntet und in einem Gamma-Counter vermessen, wodurch die Werte für den "experimental release" erhalten werden. Für die Bestimmung des "total release" werden die Zellen wie oben behandelt, wobei Serum durch eine Lösung aus 2% SDS, 50 mM Na$_2$CO$_3$ and 10 mM EDTA ersetzt wird. Die Werte für den "spontaneous release" werden erhalten, in dem Serum durch Medium A ersetzt wird. Das Resultat wird wie folgt berechnet:

$$\% \text{ Lyse} = \frac{\text{experimental release - spontaneous release}}{\text{total release - spontaneous release}} \, x100$$

Der Test wird in 3-fach Werten durchgeführt und der Mittelwert und Standardabweichung der Einzelergebnisse angegegen.

**Patentansprüche**

1.  Vakzine zur prophylaktischen und/oder therapeutischen Behandlung von Krebs enthaltend einen Antikörper, der gegen das zelluläre Membranantigen Ep-CAM gerichtet ist, in einer Menge von 0,01 bis 4 mg, und mindestens noch ein Vakzine-Adjuvans.

2.  Vakzine nach Anspruch 1 enthaltend einen Antikörper, der gegen das zelluläre Membranantigen Ep-CAM gerichtet ist, in einer Menge von 0,5 mg.

3.  Vakzine nach Anspruch 1 oder 2, wobei das Vakzine-Adjuvans ausgewählt ist aus Aluminiumhydroxid, Derivate von Lipopotysaccharid, Bacillus Calmette Guerin, Liposomen zu bereitungen und Formulierungen mit zusätzlichen Antigenen.

4.  Vakzine nach einem der Ansprüche 1 bis 3, wobei der Antikörper tierischen Ursprungs ist.

5.  Vakzine nach Anspruch 4, wobei der Antikörper murinen Ursprungs ist.

6.  Vakzine nach einem der Ansprüche 1 bis 5, wobei der Antikörper ein monoclonaler Antikörper ist.

7.  Vakzine nach einem der Ansprüche 1 bis 6, wobei der Antikörper ausgewählt ist aus der Gruppe bestehend aus intakten Antikörpern, deren Fragmenten und Derivaten mit dem Idiotyp des Ausgangs-Antikörpers.

8.  Vakzine nach einem der Ansprüche 1 bis 7, wobei zwei oder mehr Antikörper, die gegen Ep-CAM und verschiedene zelluläre Membranantigene oder gegen verschiedene Epitope von Ep-CAM gerichtet sind, in Kombination enthalten sind.

9. Vakzine nach einem der Ansprüche 1 bis 8, wobei die schwere Kette der variablen Region des Antikörpers die Aminosäuresequenz

QVQLQQSGAELVRPGTSVKVSCKASGYAFTNYLIEWVKQRPGQGLEWIG VINPGSGGTNYNEKFKGKATLTADKSSSTAYMQLSSLTSDDSAVYFCARD GPWFAYWGQGTLVTVSA (SEQ ID NO: 1)

enthätt.

10. Vakzine nach einem der Ansprüche 1 bis 9, wobei die leichte Kette der variablen Region des Antikörpers die Aminosäuresequenz

NIVMTQSPKSMSMSVGERVTLTCKASENVVTYVSWYQQKPEQSPKLLIYG ASNRYTGVPDRFTGSGSATDFTLTISSVQAEDLADYHCGQGYSYPYTFGG GTKLEIK (SEQ ID NO. 2)

enthält.

## Claims

1. A vaccine for the prophylactic and/or therapeutic treatment of cancer, containing an antibody directed against the cellular membrane antigen Ep-CAM in an amount of 0.01 to 4 mg, and at least additionally a vaccine adjuvant.

2. The vaccine according to claim 1, containing an antibody directed against the cellular membrane antigen Ep-CAM in an amount of 0.5 mg.

3. The vaccine according to claims 1 or 2 wherein the vaccine adjuvant is selected from the group consisting of aluminium hydroxide, derivatives of lipopolysaccharide, Bacillus Calmette Guerin, liposome preparations and formulations with additional antigens.

4. The vaccine according to any one of claims 1 to 3 wherein the antibody is of animal origin.

5. The vaccine according to claim 4 wherein the antibody is of murine origin.

6. The vaccine according to any one of claims 1 to 5, wherein the antibody is a monoclonal antibody.

7. The vaccine according to any one of claims 1 to 6 wherein the antibody is selected from the group consisting of intact antibodies, their fragments and derivatives with the idiotype of the initial antibody.

8. The vaccine according to any one of claims 1 to 7 containing in combination two or more antibodies, directed against Ep-CAM and different cellular membrane antigens or against different epitopes of Ep-CAM.

9. The vaccine according to any one of claims 1 to 8 wherein the heavy chain of the variable region of the antibody contains the amino acid sequence

QVQLQQSGAELVRPGTSVKVSCKASGYAFTNYLIEWVKQRPGQGLEWIG VINPGSGGTNYNEKFKGKATLTADKSSSTAYMQLSSLTSDDSAVYFCARD GPWFAYWGQGTLVTVSA (SEQ ID NO:1).

10. The vaccine according to any one of claims 1 to 9 wherein the light chain of the variable region of the antibody contains the amino acid sequence

NIVMTQSPKSMSMSVGERVTLTCKASENVVTYVSWYQQKPEQSPKLLIYG
ASNRYTGVPDRFTGSGSATDFTLTISSVQAEDLADYHCGQGYSYPYTFGG
GTKLEIK (SEQ ID NO:2).

**Revendications**

1. Vaccin pour le traitement prophylactique et/ou thérapeutique du cancer contenant un anticorps, qui est dirigé contre l'antigène membranaire cellulaire Ep-CAM, en une quantité de 0,01 à 4 mg, et au moins encore un adjuvant vaccinal.

2. Vaccin selon la revendication 1 contenant un anticorps, qui est dirigé contre l'antigène membranaire cellulaire Ep-CAM, en une quantité de 0,5 mg.

3. Vaccin selon la revendication 1 ou 2, où l'adjuvant vaccinal est choisi parmi l'hydroxyde d'aluminium, les dérivés de lipopolysaccharides, le bacille de Calmette Guérin, les préparations de liposomes et les formulations avec des antigènes supplémentaires.

4. Vaccin selon l'une des revendications 1 à 3 où l'anticorps est d'origine animale.

5. Vaccin selon la revendication 4 où l'anticorps est d'origine murine.

6. Vaccin selon l'une des revendications 1 à 5 où l'anticorps est un anticorps monoclonal.

7. Vaccin selon l'une des revendications 1 à 6 où l'anticorps est choisi dans le groupe consistant en les anticorps intacts, leurs fragments et dérivés ayant l'idiotype de l'anticorps initial.

8. Vaccin selon l'une des revendications 1 à 7 où deux ou plusieurs anticorps, qui sont dirigés contre Ep-CAM et différents antigènes membranaires cellulaires ou contre différents épitopes de Ep-CAM, sont contenus en combinaison.

9. Vaccin selon l'une des revendications 1 à 8 où la chaîne lourde de la région variable de l'anticorps contient la séquence d'acides aminés

QVQLQQSGAELVRPGTSVKVSCKASGYAFTNYLIEWVKQRPGQGLEWIG
VINPGSGGTNYNEKFKGKATLTADKSSSTAYMQLSSLTSDDSAVYFCARD
GPWFAYWGQGTLVTVSA (SEQ ID NO: 1) .

10. Vaccin selon l'une des revendications 1 à 9 où la chaîne légère de la région variable de l'anticorps contient la séquence d'acides aminés

NIVMTQSPKSMSMSVGERVTLTCKASENVVTYVSWYQQKPEQSPKLLIYG
ASNRYTGVPDRFTGSGSATDFTLTISSVQAEDLADYHCGQGYSYPYTFGG
GTKLEIK (SEQ ID NO: 2) .

## Figur 1

SW2 Zellen in Medium mit 100µg/ml HE2, Stunde 0

SW2 Zellen in Medium mit 100µg/ml HE2, Stunde 4

## Figur 2

SW2 Zellen in Medium, Stunde 0

SW2 Zellen in Medium, Stunde 4

**Figur 3**

Vakzinierung von Ziegen mit HE2-F(ab)'2:
Serum Ig mit Spezifität für HE2

**Figur 4**

## Vakzinierung von Ziegen mit HE2-F(ab)'2: Bindung von Serum Ig an Ep-CAM positive menschliche Magenkrebszellen (KATO III)

**Figur 5**

## Vakzinierung von Ziegen mit HE2-F(ab)'2: Bindung von Serum-Ig an Ep-CAM negative menschliche Melanomzellen (WM9)

## Figur 6

Vakzinierung von Ziegen mit HE2-F(ab)'2:
Bindung von affinitätsgereinigtem Serum Ig an Ep-CAM positive
menschliche Magenkrebszellen (KATO III)

**Figur 7**

### Vakzinierung von Ziegen mit HE2-F(ab)'2:
### Bindung von affinitätsgereinigtem Serum Ig an Ep-CAM negative
### menschliche Melanomzellen

## Figur 8

Vakzinierung von Rhesusaffen mit HE2
Bindung von Serum Ig an HE2

Geometrische Mittelwerte und
Standardabweichung der
Ergebnisse von 4 Rhesusaffen

**Figur 9**

Vakzinierung von Rhesusaffen mit HE2
Bindung von Serum Ig an Kato III Tumorzellen

**Figur 10**

## Rhesusaffenstudie
## Antikörpertiter gegen HE2

Rezlproker Titer bei OD=1

Tag

HE2    Placebo    HE2    Placebo    HE2    Placebo

**Figur 11**

## Rhesusaffenstudie:
## Serum Ig gegen Kato III Tumorzellen

Legend:
- 9599m d0
- 9599m d29
- 9139f d0
- 9139f d29
- HE2 100ug/ml

X-axis: Serumverdünnung 1:
Y-axis: OD x 1000

**Figur 12**

## Vakzinierung eines Darmkrebspatienten mit HE2: Induktion von Antikörpern gegen menschliche Magenkrebszellen (KATO III)

Serum titer, arbiträre Einheiten

s.c. Vakzinierung mit 0.5 mg HE2 auf Al(OH)3

Präserum  Tag 15  Tag 50  Tag 64  Tag 78  Tag114  Tag 128

## Figur 13

### Vakzinierung eines Darmkrebspatienten mit HE2: Induktion von Serum-Zytotoxizität gegen menschliche Magenkrebszellen (KATO III)

SEQUENZPROTOKOLL

<110> IGENEON AG

<120> Verwendung von Antikörpern zur Vakzinierung gegen Krebs

<130> D 2923 PCT

<140>
<141>

<160> 2

<170> PatentIn Ver. 2.1

<210> 1
<2.11> 116
<212> PRT
<213> Mus musculus

<400> 1

```
Gln Val Gln Leu Gln Gln Ser Gly Ala Glu Leu Val Arg Pro Gly Thr
  1               5                  10                  15

Ser Val Lys Val Ser Cys Lys Ala Ser Gly Tyr Ala Phe Thr Asn Tyr
            20                  25                  30

Leu Ile Glu Trp Val Lys Gln Arg Pro Gly Gln Gly Leu Glu Trp Ile
            35                  40                  45

Gly Val Ile Asn Pro Gly Ser Gly Gly Thr Asn Tyr Asn Glu Lys Phe
        50                  55                  60

Lys Gly Lys Ala Thr Leu Thr Ala Asp Lys Ser Ser Ser Thr Ala Tyr
 65                  70                  75                  80

Met Gln Leu Ser Ser Leu Thr Ser Asp Asp Ser Ala Val Tyr Phe Cys
                85                  90                  95

Ala Arg Asp Gly Pro Trp Phe Ala Tyr Trp Gly Gln Gly Thr Leu Val
            100                 105                 110

Thr Val Ser Ala
            115
```

<210> 2

<211> 107
<212> PRT
<213> Mus musculus

<400> 2

```
Asn Ile Val Met Thr Gln Ser Pro Lys Ser Met Ser Met Ser Val Gly
 1               5                  10                  15

Glu Arg Val Thr Leu Thr Cys Lys Ala Ser Glu Asn Val Val Thr Tyr
            20                  25                  30

Val Ser Trp Tyr Gln Gln Lys Pro Glu Gln Ser Pro Lys Leu Leu Ile
            35                  40                  45


Tyr Gly Ala Ser Asn Arg Tyr Thr Gly Val Pro Asp Arg Phe Thr Gly
        50                  55                  60

Ser Gly Ser Ala Thr Asp Phe Thr Leu Thr Ile Ser Ser Val Gln Ala
 65                  70                  75                  80

Glu Asp Leu Ala Asp Tyr His Cys Gly Gln Gly Tyr Ser Tyr Pro Tyr
                85                  90                  95

Thr Phe Gly Gly Gly Thr Lys Leu Glu Ile Lys
            100                 105
```